# EUROPEAN PATENT APPLICATION

(11) **EP 4 246 145 A1**
(43) Date of publication of application: **20.09.2023**
(21) Application number: 21891091.7
(22) Date of filing: 09.11.2021
(51) Int. Cl.: G01N 33/53, G01N 21/78, G01N 21/01

(54) **TEST DEVICE AND TEST PAPER**

(30) Priority: 10.11.2020 CN 202011247527
(71) Applicant: Leadway (HK) Limited, Sheung Wan, Hong Kong (CN)
(72) Inventor: CAI, Zhengjun, Hangzhou, Zhejiang 310030 (CN); SHANG, Tao, Hangzhou, Zhejiang 310030 (CN); FEI, Fengqin, Hangzhou, Zhejiang 310030 (CN); HU, Haibin, Zhejiang 310030 (CN); JI, Pinlan, Hangzhou, Zhejiang 310030 (CN); KONG, Jianxi, Hangzhou, Zhejiang 310030 (CN); TONG, Fangli, Hangzhou, Zhejiang 310030 (CN); CHU, Jiaoyan, Hangzhou, Zhejiang 310030 (CN); LI, Yanqun, Hangzhou, Zhejiang 310030 (CN); DU, Yujiao, Hangzhou, Zhejiang 310030 (CN)
(74) Representative: Schiweck Weinzierl Koch Patentanwälte Partnerschaft mbB
(86) International application number: PCT/CN2021/129559
(87) International publication number: WO 2022/100571

(57) **Abstract**

A detection device includes a test cassette and a test strip, wherein the test strip is mounted between the upper cover and bottom plate of the test cassette, a positioning column is disposed in the test cassette, and a positioning hole is formed in the test strip. The longitudinal dimension of the positioning hole is basically the same as that of the positioning column, and the transverse dimension of the positioning hole is larger than that of the positioning column. The present invention further provides the test strip with the positioning hole, and through the mutual cooperation relationship of the positioning column in the test cassette and the positioning hole in the test strip, the success rate of mounting the test strip to the test cassette can be improved, and the scrap rate of production of the test strip can be reduced.

## Description

### Field of the Invention

The present invention relates to the detection field using test strip for detection, in particular to test strip and a test cassette for placement of said test strip.

### Background of the Invention

At present, more and more products capable of achieving in situ sampling and obtaining detection results immediately are being investigated and used in clinical applications, and such detections are generally referred to as point-of-care testing (POCT).

Such POCT is usually carried out using dry test strip with a reagent. For example, the lateral flow test strip generally includes a supporting pad, on which a sample addition pad, a label pad, a detection pad and a water absorbing pad are disposed in a manner of being superposed on each other in sequence from upstream to downstream. A liquid sample added to the sample addition pad flows past the sample addition pad, the label pad, the detection pad and the water absorbing pad sequentially. After the detection is completed, the gradation of the color shown on the detection pad is linearly correlated with the concentration of the analyte in the sample to be detected, thereby determining the level of the corresponding analyte in the sample. For example, vertical flow test strip generally includes a supporting pad, on which a sample pad, a separation pad and a detection pad are disposed in sequence from top to bottom, the supporting pad being provided with an observation hole at the detection pad. The test strip also includes various forms of test strip such as urine combination test strip which is provided with a plurality of detection pads spaced on the supporting pad. The test strip can be used to measure hormone levels, drug types, allergens, physiological indicators in urine, HBV, HCV, HIV, malaria, dengue fever, influenza, Middle East Respiratory Syndrome, SARS and 2019-nCoV, blood glucose, uric acid, blood ketone, cholesterol, lactic acid, etc. by using various detection methods such as electrochemical methods and photochemical methods that are suitable for the requirements of POCT.

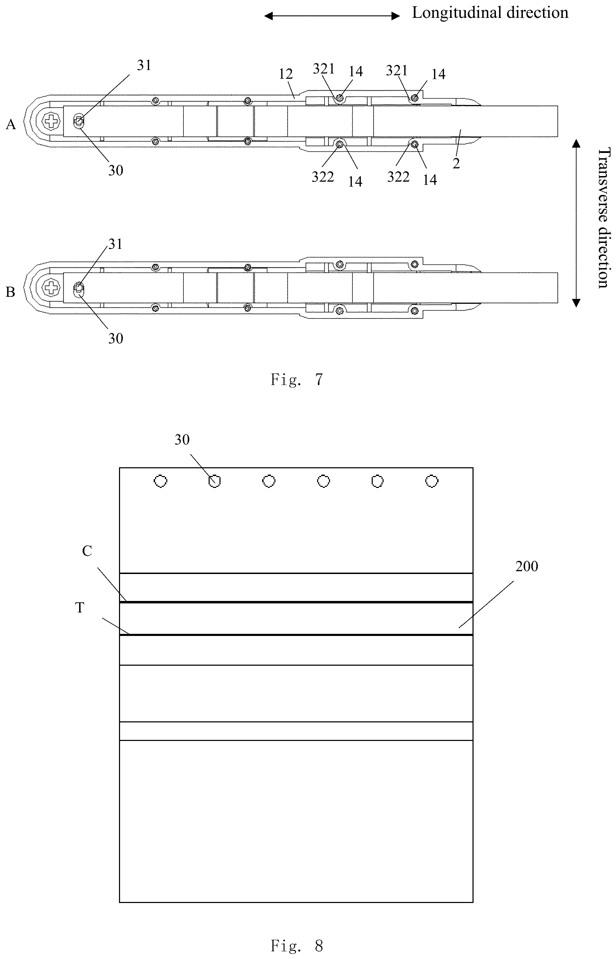

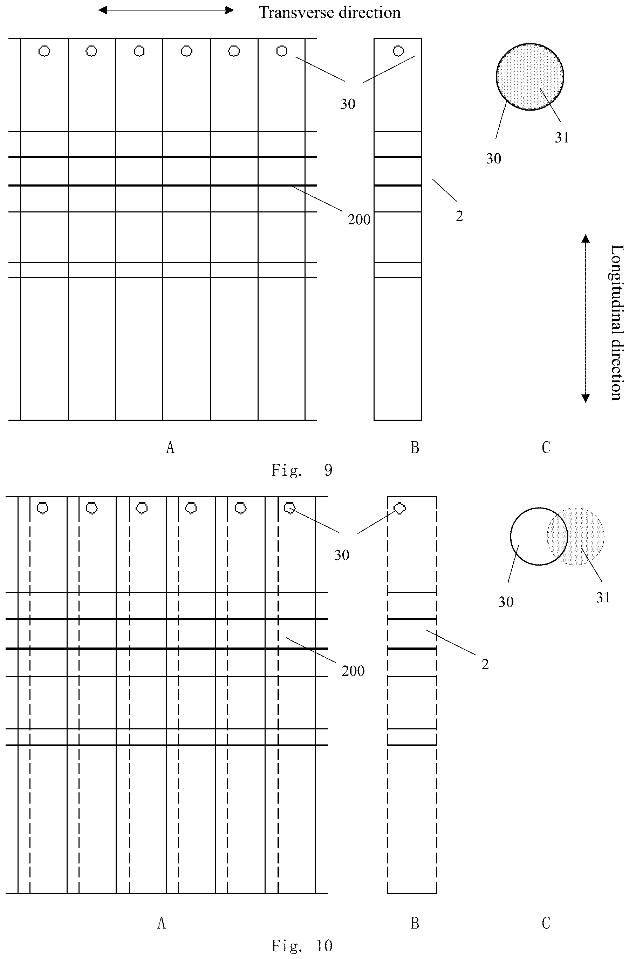

As shown in Figs. 8 and 9, the sample addition pad, the label pad, the detection pad containing a test line (T line) and a control line (C line), the water absorbing pad, etc. of the test strip are generally made on a large card 200 in advance, a positioning hole 30 is punched in the position of the water absorbing pad on the large card, and then the large card is cut in the longitudinal direction into single pieces of test strip by using hobs or other cutters. The test strip can be directly used for detection, or the test strip can be mounted in the test cassette. The test cassette generally includes an upper cover and a bottom plate, and the test strip is mounted between the upper cover and the bottom plate, and the upper cover or the bottom plate is provided with a sample addition hole and a test result reading window. In order that the test strip mounted in the test cassette does not generate longitudinal movement and deviate from the mounting position, a positioning column 31 is usually disposed on the inner wall of the test cassette, and a positioning hole 30 is formed in the corresponding position on the test strip. When the test strip is assembled in the test cassette, the test strip is placed on the positioning column through the positioning hole to prevent the test strip from moving inside the test cassette as much as possible.

The shape of the positioning hole in the existing products is the same as that of the positioning column, for example, if the positioning column is round, the positioning hole is also round, and the positioning column can be exactly inserted into the positioning hole to achieve a tight fit or interference fit of the positioning column and the positioning hole in both the longitudinal and transverse directions. In such a design solution, the position of the positioning hole in the test strip must be consistent with the position of the positioning column, for example, if the center point of the positioning column is on the longitudinal central axis of the detection device, then the center point of the positioning hole must also be on the longitudinal central axis of the test strip. Solid longitudinal lines on the large card 200 as shown in Fig. 9A indicate the exact cutting positions, and a single piece of test strip 2 as shown in Fig. 9B is obtained by cutting according to the solid line positions, with the center point of the positioning hole 30 of the single piece of test strip being located exactly on the longitudinal central axis of the test strip. When a hob is accurately cutting in a solid line position, the round positioning column 31 as shown in Fig. 9C can be inserted into the round positioning hole 31 of the test strip.

If the position of the positioning hole deviates, such as in the case of positioning hole deviation resulted from crooked cutting of a hob or cutter, the positioning column of the test cassette cannot be inserted into the positioning hole of the test strip, and thus the test strip cannot be mounted in the test cassette.

As shown in Fig. 10A, the longitudinal solid lines on the large card 200 indicate the accurate cutting positions, and the longitudinal dashed lines indicate the actual cutting positions of the hob, and in this example, the cutting positions (dashed lines) are offset to the right than the preset positions (solid lines). The hob performs cutting according to the dashed line positions to obtain single pieces of test strip 2 as shown in Fig. 10B, and the positioning hole 30 in a single piece of test strip deviates from the longitudinal central axis to the left. This results in that as shown in Fig. 10C, the round positioning column 31 can not be inserted into the round positioning hole 30 of the test strip.

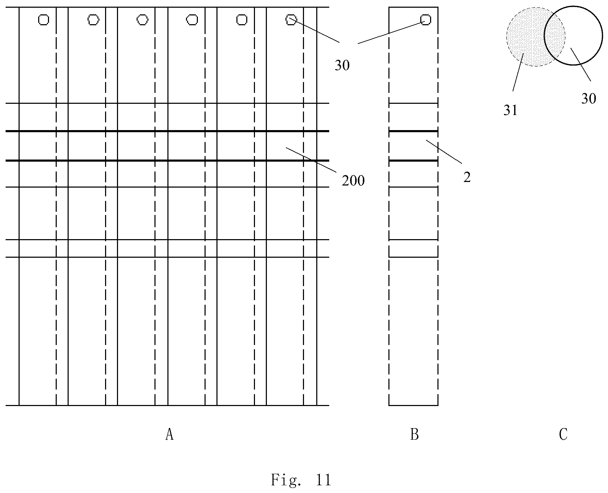

As shown in Fig. 11A, the longitudinal solid lines on the large card 200 indicate the accurate cutting positions, and the longitudinal dashed lines indicate the actual cutting positions of the hob, and in this example, the cutting positions (dashed lines) are offset to the left than the preset (solid lines) positions. The hob performs cutting according to the dashed line positions to obtain single pieces of test strip 2 as shown in Fig. 11B, and the positioning hole 30 in the single piece of test strip deviates from the longitudinal central axis to the right. This results in that as shown in Fig. 11C, the round positioning column 31 can not be inserted into the round positioning hole 30 of the test strip. Therefore, in the manufacturing process, the existing processing technology of the test strip will lead to a higher scrap rate of the test strip, and the test strip shown in Figs. 10B and 11B cannot be mounted to the test cassette for use. Therefore, each time the test strip is made, the position of the positioning hole must be strictly controlled during product design. If a hob, a cutter or the like carries out the cutting operation, it will cause the positioning hole to slightly deviate from the pre-designed position in the case of crooked cutting. When the positioning column is inserted into the positioning hole of such test strip, the test strip cannot be mounted at the correct position on the detection device because the positioning hole deviates from a preset position, and the detection cannot be carried out. Therefore, there's a high requirement for the precision of the production process of the current test strip with a positioning hole.

### Summary of the Invention

In order to improve the success rate of mounting test strip to a test cassette in the case of reducing the precision of the production process of the test strip, especially the cut precision of the test strip, one of the objectives of the present invention is to provide a detection device. The detection device comprises a test cassette and test strip; the test cassette comprises an upper cover and a bottom plate, the test strip is mounted between the upper cover and the bottom plate, a positioning column is disposed in the test cassette, and a positioning hole is formed in the test strip. Said positioning column is inserted into the positioning hole, the longitudinal dimension of the positioning hole is basically the same as that of the positioning column, and the dimension of the positioning hole in other directions, especially in the transverse direction, is equal to or larger than the dimension of the positioning column. When the dimension in other directions is equal to or larger than the dimension of the positioning column, it is easier to insert the positioning column into the positioning hole of such test strip, so as to prevent the fact that the test strip cannot be mounted at a correct position on the detection device because the positioning hole deviates from a preset position.

Further, as for the cooperation of said positioning hole and the positioning column, in the longitudinal direction, the positioning hole and the positioning column are in tight fit; and in other directions, especially in the transverse direction, the positioning hole and the positioning column are in loose fit.

The shape of said positioning column is round, square, rectangular or cross-shaped. Accordingly, said positioning hole is oval or strip-shaped or rectangular. The shape of said positioning hole corresponding to the positioning column is oval or rectangular.

Preferably, the positioning column is located on the longitudinal central axis of the test cassette, and the central point of said positioning hole is located near by the central axis of the test strip.

Further, the test cassette also comprises a transverse limiting column. Preferably, at least two transverse limiting columns are comprised, which are located on both sides of the longitudinal central axis of the test cassette, respectively; or further, said two transverse limiting columns are equidistantly distributed on both sides of the longitudinal central axis of the test cassette. The distance between the transverse limiting columns on the two opposite sides achieves a tight fit between the test strip placed therein and the transverse limiting columns.

The present invention further provides test strip, which comprises a supporting pad, a detection pad disposed on the supporting pad and a positioning hole, wherein the positioning hole is used to accommodate the positioning column, the longitudinal dimension of the positioning hole is basically the same as that of the positioning column, and the transverse dimension of the positioning hole is larger than that of the positioning column.

Test strip in the form of lateral flow includes a supporting pad, on which a sample addition pad, a label pad, a detection pad and a water absorbing pad are disposed in a manner of being superimposed on each other in sequence from upstream to downstream. The positioning hole passes through the water absorbing pad.

Test strip comprises a supporting pad, a detection pad disposed on the supporting pad, or a plurality of detection pads, which are spaced on the supporting pad. The positioning hole is located on the supporting pad.

The transverse dimension of the positioning hole in the test strip is larger than that of the positioning column of the test cassette, so that loose fit is formed between the positioning hole and the positioning column in the transverse direction, which can effectively reduce the probability that a single piece of test strip cut out of alignment can not be mounted in the test cassette due to the deviation generated when a large card is cut to manufacture the single piece of test strip, and reduce the scrap rate of materials. Through effective cooperation of the positioning hole and the positioning column, the test strip is stably held at a preset position on the test cassette; and meanwhile, the phenomenon of twist of the test strip generated after the test strip cut obliquely is mounted in the test cassette can be greatly avoided to ensure the accuracy of detections.

### Brief Description of the Drawings

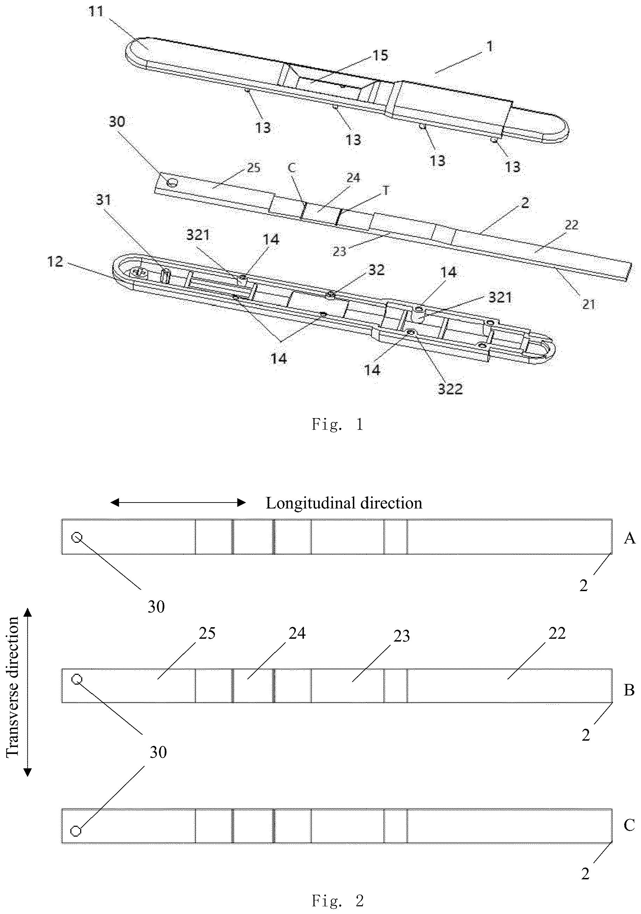

Fig. 1 is an exploded schematic diagram of a detection device containing test strip of the present invention.
Fig. 2 is test strip with a round positioning hole, with test strip A indicating that the round positioning hole is located on the central axis of the test strip, test strip B indicating that the round positioning hole deviates above the central axis, and test strip C indicating that the round positioning hole deviates below the central axis.

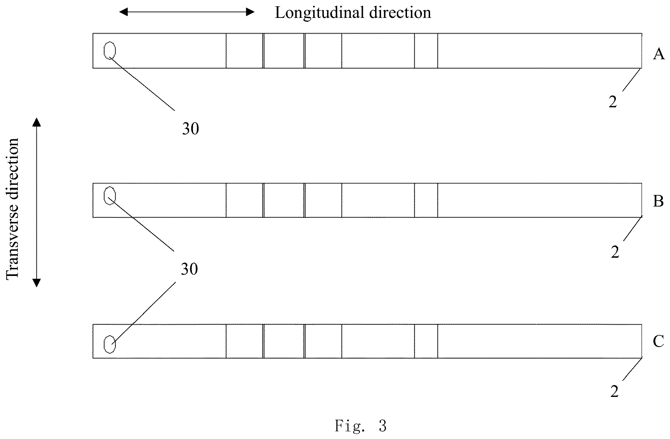

Fig. 3 is test strip with an oval positioning hole, with test strip A indicating that the oval positioning hole is located on the central axis of the test strip, test strip B indicating that the oval positioning hole deviates above the central axis, and test strip C indicating that the oval positioning hole deviates below the central axis.

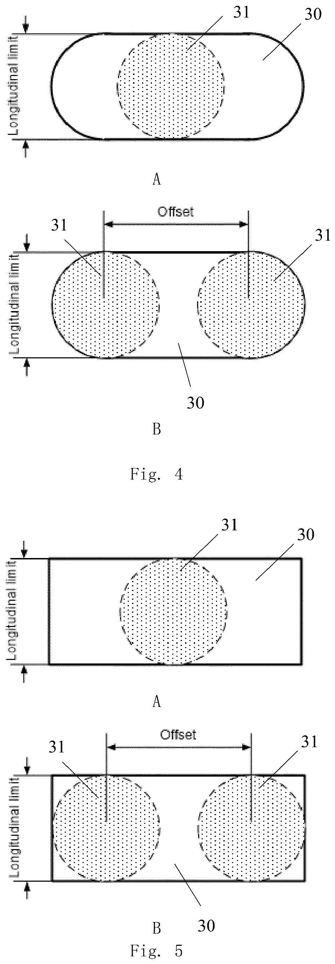

Fig. 4 shows the position relation of an oval positioning hole and a round positioning column. A indicates that the positioning column is in the middle of the positioning hole, and B indicates that the positioning column is offset to the right or left of the positioning hole.
Fig. 5 shows the position relation of a rectangular positioning hole and a round positioning column. A indicates that the positioning column is in the middle of the positioning hole, and B indicates that the positioning column is offset to the right or left of the positioning hole.

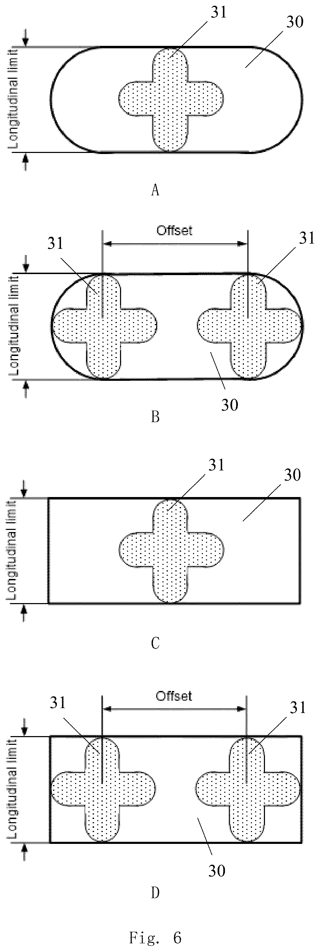

Fig. 6 shows the position relation of an oval or rectangular positioning hole and a cross-shaped positioning column. A and C indicate that the positioning column is in the middle of the positioning hole, and B and D indicate that the positioning column is offset to the right or left of the positioning hole.
Fig. 7 is a schematic diagram of mounting the test strip of the present invention to a bottom plate. A indicates that the positioning hole of the test strip is located exactly on the central axis of the test strip, and the positioning column inserted into the positioning hole is located in the middle of the positioning hole. B indicates that the positioning hole of the test strip is not formed in alignment and located at a position below the central axis of the test strip, the positioning column can still be inserted into the positioning hole accurately, and the positioning column is located on the side of the positioning hole.
Fig. 8 is an uncut large card.
Fig. 9 is an example that a hob performs cutting at an accurate position on the large card. 9A indicates an uncut large card, and the solid line in the longitudinal direction of the large card indicates the correct cutting position; 9B indicates a single piece of cut test strip; and 9C indicates the case that the positioning column is inserted into the positioning hole of the test strip in 9B.
Fig. 10 is an example that the cutting position of the hob deviates from the accurate cutting position to the right; 10A indicates an uncut large card, the solid line in the longitudinal direction of the large card indicates the correct cutting position, and the dashed line indicates the actual cutting position;
10B indicates a single piece of cut test strip; and 10C indicates the case that the positioning column cannot be inserted into the positioning hole of the test strip in 10B.
Fig. 11 is an example that the cutting position of the hob deviates from the accurate cutting position to the left; 11A indicates an uncut large card, the solid line in the longitudinal direction of the large card indicates the correct cutting position, and the dashed line indicates the actual cutting position; 11B indicates a single piece of cut test strip; and 11C indicates the case that the positioning column cannot be inserted into the positioning hole of the test strip in 11B.

### Detailed Description of the Embodiments

The present invention will be described below in detail in conjunction with specific drawings. These specific embodiments are only limited enumeration without departing from the spirit of the present invention, and do not exclude other specific embodiments derived from the combination of the prior art and the present invention by a person of ordinary skill in the art.

As shown in Fig. 1, The detection device 1 includes a test cassette and test strip; the test cassette includes an upper cover 11 and a bottom plate 12; and the upper cover and the bottom plate are assembled together to become the housing of the test cassette. Test strip 2 is mounted between the upper cover and the bottom plate, and the test strip is placed at a relatively fixed position within the test cassette to ensure that the detection result can be accurately captured. A round positioning column 31 is designed at one end of the bottom plate, and is disposed on the longitudinal central axis of the detection device. A positioning hole 30 is formed in the test strip at a position corresponding to the positioning column, and the positioning column 31 is inserted into the positioning hole 30 to reduce or eliminate the movement of the test strip in the longitudinal direction of the test cassette. Transverse limiting columns 32 are also designed on both sides of the bottom plate relative to the longitudinal direction of the test strip, and the distance between the transverse limiting columns 321 and 322 on the two opposite sides of the bottom plate allows the test strip placed between the two transverse limiting columns to be in clamping fit with the transverse limiting columns to reduce or eliminate the movement of the test strip in the transverse direction of the test cassette; or the limiting columns on both sides of the bottom plate limit the movement magnitude of the test strip in the transverse direction of the bottom plate, so as to ensure that the detection area on the test strip can be displayed in the detection window of the test cassette. The positioning column and the transverse limiting columns can be disposed on the bottom plate as separate elements or in combination with other elements on the bottom plate. The positioning column and the limiting columns can also be disposed on the upper cover.

Rivets 13 are disposed around the inner side of the upper cover, and locking holes 14 are formed in the bottom plate at positions corresponding to the rivets on the upper cover, and the rivets 13 on the upper cover and the locking holes 14 in the bottom plate are in interference fit to assemble the upper cover and the bottom plate tightly together. Of course, positions of the rivets and the locking holes are interchangeable on the upper cover and the bottom plate. In a preferred design, said locking holes 14 are formed in the transverse limiting columns 32. It is also possible that no rivets and locking holes are disposed between the upper cover and the bottom plate, they can also be assembled together by other means such as ultrasonic welding.

The longitudinal dimension of the positioning hole 30 of the test strip and the longitudinal dimension of the positioning column 31 of the test cassette are in tight fit, such as a mutual clamping or interference fit, and the positioning column basically cannot move in the longitudinal direction of the positioning hole, thus limiting the movement of the test strip in the longitudinal direction of the test cassette. The transverse dimension of the positioning hole 30 of the test strip is larger than the transverse dimension of the positioning column 31 of the test cassette, and the positioning column can move in the transverse direction of the positioning hole. The movement of the test strip in the transverse direction of the test cassette is limited by the transverse limiting columns of the test cassette.

In the process of cutting single pieces of test strip or punching out positioning holes, it may result in different positions of the positioning holes in different single pieces of test strip, for example, the positioning hole is located exactly on the longitudinal central axis of the test strip, as shown by A of Fig. 2 and A of Fig. 3; the positioning hole is located above the longitudinal central axis, as shown by B of Fig. 2 and B of Fig. 3; and the positioning hole is located below the longitudinal central axis, as shown by C of Fig. 2 and C of Fig. 3. The positioning holes in A of Fig. 2 to C of Fig. 2 belong to the prior art, the positioning hole cooperating with the round positioning column is round, and there is a clamping fit between the longitudinal and transverse dimensions of the positioning hole 30 in the test strip and the longitudinal and transverse dimensions of the round positioning column 31 of the detection device. The positioning holes in A in Fig. 3 to C in Fig. 3 belong to the art described in the present invention, the positioning hole cooperating with the round positioning column 31 is oval, which is formed by two edges parallel to each other and edges in the shape of arc at two ends, there is a clamping fit between the longitudinal dimension of the positioning holes 30 of the test strip and the longitudinal dimension of the round positioning columns 31 of the detection device, and the transverse dimension of the positioning hole 30 is larger than the transverse dimension of the positioning column 31 of the detection device.

When the positioning column is located on the longitudinal central axis of the test cassette and fixed, the transverse limiting columns are equidistantly distributed on both sides of the longitudinal central axis of the test cassette, then the round positioning column can still be inserted into the positioning hole after the test strip in A of Fig. 2 and A, B and C of Fig. 3 is placed between the transverse limiting columns. However, after said test strip in B and C of Fig. 2 is placed between the transverse limiting columns, the round positioning column cannot be inserted into the positioning hole where the deviation occurs.

As shown in Fig. 4, the positioning column is round and the positioning hole is oval, the longitudinal dimension of this positioning hole is basically the same as the outer diameter of the round positioning column, and the transverse dimension of the positioning hole is larger than the outer diameter of the round positioning column. The positioning column can be located in the center of the positioning hole, see A of Fig. 4, or in any position between the two ends, see B of Fig. 4.

As shown in Fig. 5, the positioning column is round and the positioning hole is rectangular, the longitudinal dimension of this positioning hole is basically the same as the outer diameter of the round positioning column, and the transverse dimension of the positioning hole is larger than the outer diameter of the round positioning column. The positioning column can be located in the center of the positioning hole, see A of Fig. 5, or in any position between the two ends, see B of Fig. 5. As shown in Fig. 6, the positioning column is cross-shaped and the positioning hole is oval or rectangular, the longitudinal dimension of this positioning hole is basically the same as the outer diameter of the cross-shaped positioning column, and the transverse dimension of the positioning hole is larger than the outer diameter of the cross-shaped positioning column. The positioning column can be located in the center of the positioning hole, see A and C of Fig. 6, or in any position between the two ends, see B and D of Fig. 6.

As shown in A of Fig. 7, the cross-shaped positioning column is located on the longitudinal central axis of the test cassette, the center point of the oval positioning hole of a single piece of test strip is to be located exactly on the longitudinal central axis of the test strip, and then after the test strip is placed between the transverse limiting columns, the cross-shaped positioning column can be inserted into the positioning hole. As shown in B of Fig. 7, the position of the positioning hole of a single piece of test strip deviates, and the center point of the positioning hole is not located on the longitudinal central axis of the test strip. After the test strip is placed between the transverse limiting columns, the cross-shaped positioning column can be inserted in a position close to the top in the positioning hole, so that the cross-shaped positioning column can still be inserted into the positioning hole smoothly. When the test strip is mounted in place, both the sample addition pad and the detection pad are at accurate positions on the detection device, ensuring that detection is smooth and the detection result in the detection area can be obtained smoothly from the detection window 15 of the detection device. Even if position deviation of the positioning hole occurs in the machining process, the technology of the present invention still can allow the test strip to be mounted at the correct position within the detection device, and the cooperation of the positioning hole and the positioning column described in the present invention can reduce or limit the longitudinal movement of the test strip in the detection device, and the cooperation of the transverse limiting columns and both sides of the test strip can limit the transverse movement of the test strip in the detection device.

The test strip can be lateral flow test strip 2 as shown in Fig. 1, including a supporting pad 21, a sample addition pad 22, a label pad 23, a detection pad 24 and a water absorbing pad 25, the label pad being provided with a test line T and a control line C, the positioning hole being at the position of the water absorbing pad. Said test strip can be used to detect hormones such as HCG, LH and FSH, drugs, HBV, HCV, HIV, malaria, dengue fever, influenza, Middle East respiratory syndrome, SARS and 2019-nCoV, etc.

Another type of test strip includes a supporting pad and a detection pad disposed on the supporting pad, the sample to be detected is directly added to the sample addition pad, and the positioning hole is directly formed in the supporting pad. Said test strip can be used to detect blood glucose, cholesterol, triglycerides, glutathione transaminase, etc.

The test strip includes a supporting pad and different types of detection pads spaced on the supporting pad. For various forms of test strip such as urine combination test strip, the positioning hole may also be disposed in the supporting pad.

The detection items of the test strip can be designed according to actual needs. The detection items as described in the present invention are merely illustration, instead of limit to the type and detection items of the test strip.

Said positioning hole is used to accommodate the positioning column, for example, when the test strip is mounted in the test cassette, the positioning column in the test cassette is inserted into said positioning hole. The person skilled in the art can design a positioning column and a positioning hole that are different in shape as needed, and the positioning hole reserves a certain offset for the positioning column in the longitudinal direction. The final dimension of the positioning hole in the transverse direction can also be enlarged as much as possible on the basis of taking the width and strength of the test strip into account and meeting the structural strength. The positioning hole is disposed in a corresponding appropriate position on the test strip according to different positions of the positioning column.

In the present invention, when the test strip in the manner of lateral flow is used, the length direction of said test strip is defined as the longitudinal direction, i.e., the direction of liquid flowing on the test strip from the sample addition pad to the water absorbing pad is the longitudinal direction, and the direction perpendicular to the liquid flow direction is the transverse direction. Alternatively, the longitudinal and transverse directions described in the present invention may also be defined in the following manner: as shown in Fig. 9, with the cutting line as a reference, in the process of cutting the large card into single pieces of test strip, the direction parallel to the cutting line of the cutter is the longitudinal direction, and the direction perpendicular to the cutting line is the transverse direction. Alternatively, the longitudinal and transverse directions described in the present invention may also be determined as follows: with the transverse limiting columns of the test cassette as references, which two transverse limiting columns confine the test strip in the test cassette, The direction of the relative connecting line between the two opposed transverse limiting columns is transverse direction, and the direction perpendicular to the direction of the relative connecting line is the longitudinal direction; and specifically as shown in Fig. 7A, the direction of the relative connecting line of the right limiting column 321 and the left limiting column 322 is the transverse direction.

The loose fit of the positioning hole and the positioning column in the transverse direction described in the present invention reduces the probability that the test strip cannot be assembled in the test cassette because of the deviation arising from manufacturing single pieces of test strip by cutting the large card and consequent deviation of the positioning hole. It also can reduce the probability that the test strip cannot be assembled in the test cassette because the positioning hole and the positioning column can not be assembled due to the deviation of the positioning hole generated when punching out the positioning hole. The design solution of the present invention can reduce the scrap rate of materials in the process of manufacturing the test strip. During mounting, it reduces the situation that the distortion of the test strip in the detection device resulted from deviation of the test strip, which affects the test result.

## Claims

1. A detection device, comprising a test cassette and test strip, the test cassette comprising an upper cover and a bottom plate, the test strip being mounted between the upper cover and the bottom plate, a positioning column being disposed in the test cassette, said positioning column being inserted into the positioning hole, wherein the positioning hole and the positioning column are in clamping fit in the longitudinal direction, and the positioning hole and the positioning column are in loose fit in the transverse direction.

2. The detection device according to claim 1, wherein the longitudinal dimension of the positioning hole is substantially the same as the longitudinal dimension of the positioning column, and the transverse dimension of the positioning hole is larger than the transverse dimension of the positioning column.

3. The detection device according to claim 1, wherein the shape of the positioning column is round, square, rectangular or cross-shaped.

4. The detection device according to claim 3, wherein the shape of the positioning hole is oval or rectangular.

5. The detection device according to any of claims 1 to 3, wherein the positioning column is located on the longitudinal central axis of the test cassette.

6. The detection device according to claim 5, wherein the center point of said positioning hole is located near by the central axis of the test strip.

7. The detection device according to claim 5, wherein the test cassette further comprises a transverse limiting column.

8. The detection device according to claim 7, comprising at least two transverse limiting columns, said two transverse limiting columns being located on two sides of the longitudinal central axis of the test cassette.

9. The detection device according to claim 8, wherein said two transverse limiting columns are equidistantly distributed on both sides of the longitudinal central axis of the test cassette.

10. The detection device according to one of claims 8 to 9, wherein the distance between the transverse limiting columns on the two opposite sides achieves a tight fit between the test strip placed therein and the transverse limiting columns.

11. The detection device according to claim 1, wherein the test strip comprises a supporting pad and a detection pad.

12. The detection device according to claim 11, wherein the test strip comprises a supporting pad, and a sample addition pad, a label pad, a detection pad and a water absorbing pad which are placed on the supporting pad in a manner of being superimposed on each other in sequence from upstream to downstream.

13. The detection device according to claim 12, wherein the positioning hole passes through the water absorbing pad.

14. A test strip, comprising a supporting pad, a test pad and a positioning hole disposed on the supporting pad, wherein the positioning hole is used to accommodate a positioning column, the positioning hole and the positioning column are in clamping fit in the longitudinal direction, and the positioning hole and the positioning column are in loose fit in the transverse direction.

15. The test strip according to claim 14, wherein the longitudinal dimension of the positioning hole is substantially the same as the longitudinal dimension of the positioning column, and the transverse dimension of the positioning hole is larger than the transverse dimension of the positioning column.

16. The test strip according to claim 15, wherein the shape of the positioning hole is oval or rectangular.

17. The test strip according to any of claims 14 to 16, wherein the test strip comprises a supporting pad and a detection pad.

18. The test strip according to claim 17, wherein the test strip comprises a supporting pad, and a sample addition pad, a label pad, a detection pad and a water absorbing pad which are placed on the supporting pad in a manner of being superimposed on each other in sequence from upstream to downstream.

19. The test strip according to claim 18, wherein the positioning hole passes through the water absorbing pad.
